# EUROPEAN PATENT SPECIFICATION

(11) **EP 1 722 812 B1**
(45) Date of publication and mention of the grant of the patent: **13.08.2014**
(21) Application number: 05758644.8
(22) Date of filing: 17.02.2005
(51) Int. Cl.: A61K 38/43, A61K 38/46, C12P 7/06, C12P 7/08, C12P 1/02, C12P 1/04, C12P 1/06, C12N 9/00, C12N 9/30, C12N 9/54, C12N 9/62, C12P 19/14

(54) **LIQUEFACTION PROCESSES**
VERFLÜSSIGUNGSVERFAHREN
PROCEDES DE LIQUEFACTION

(30) Priority: 19.02.2004 US 546118 P
(43) Date of publication of application: 22.11.2006
(73) Proprietor: Novozymes North America, Inc., Franklinton, NC 27525 (US)
(72) Inventor: HENDERSON, Lori, Raleigh, NC 27615 (US); COSTABLE, Carmen, West Henrietta, NY 14586 (US)
(74) Representative: Kofoed, Gertrud Sonne
(86) International application number: PCT/US2005/005078
(87) International publication number: WO 2005/086640

(56) References cited:
- WO-A2-02/074895
- DE-A1- 4 125 971
- US-A1- 2002 006 647
- US-A1- 2004 063 184
- US-A1- 2004 253 696
- OSMAN A M: "The advantages of using natural substrate-based methods in assessing the roles and synergistic and competitive interactions of barley malt starch-degrading enzymes", JOURNAL OF THE INSTITUTE OF BREWING, vol. 108, no. 2, 2002, pages 204-214, XP001526002, ISSN: 0046-9750
- HAGENIMANA V ET AL: "Sweetpotato alpha- and beta-Amylases: Characterization and Kinetic Studies with Endogenous Inhibitors", JOURNAL OF FOOD SCIENCE, vol. 59, no. 2, March 1994 (1994-03), pages 373-377, XP002009050,

## Description

### FIELD OF THE INVENTION

The present invention relates to an improved process of liquefying starch-containing material suitable as a step in processes for producing syrups and fermentation products. The invention also relates to processes for producing ethanol comprising liquefying starch-containing starting material in accordance with the invention.

### BACKGROUND OF THE INVENTION

Liquefaction is a well known process in the art by which starch is converted to shorter chains and less viscous dextrins. The process generally involves gelatinization of starch simultaneously with or followed by addition of alpha-amylase. Liquefaction is used in processes for producing syrups and fermentation products, such as ethanol. There is a need for improving the liquefaction step for converting starch into syrups and fermentation products such as especially ethanol.

### SUMMARY OF THE INVENTION

The object of the present invention is to provide improved processes of liquefying starch-containing material, especially starch-containing material reduced in size by, e.g., dry milling.

The present inventors have found that liquefaction of dry milled starch-containing material may be improved by treating with at least one alpha-amylase and a maltogenic amylase or alternatively with at least one amylase and at least one esterase. The esterase is believed to attack lipids present in the starch-containing material to product smaller molecules that are less likely to produce starch-lipid complexes referred to as retrograded starch. One advantage of a process of the invention is that it improves liquefaction by reducing the viscosity of the gelatinized hot or warm slurry and prevents or at least reduces the formation of retrograded starch created during jet cooking. Further, according to the invention more carbohydrate is liberated from the raw starch-containing starting material.

Thus, in the first aspect the invention provides a process for liquefying starch-containing material comprising the step of treating said starch-containing material with at least one alpha-amylase and a maltogenic amylase.

In a second aspect the invention provides a process for liquefying starch-containing material comprising the step of treating said starch-containing material with at least one amylase and at least one esterase.

In one embodiment the liquefaction process comprises the steps of:
i) pre-treating a slurry of starch-containing material with at least one esterase,
ii) liquefying the pre-treated slurry with an alpha-amylase.

In a preferred embodiment the starch-containing material is reduced in size, preferably by dry milling. The liquefaction step ii) may be carried out as a multi-stage hot slurry process, such as a three stage process, carried out at different temperatures and holding times.

In the production of fermentation products, such as ethanol, and other starch-based products, such as syrups, the starch-containing raw material, such as whole grains, preferably corn, may be reduced in size, preferably by dry milling in order to open up the structure and allow for further processing. Techniques for reducing the size of starch-containing material, including dry milling, are well known in the art.

In another aspect the invention provides processes for producing fermentation products, such as ethanol, comprising:
(a) reducing the size of starch-containing material
(b) liquefying the product of step (a) with at least one alpha-amylase and at least one maltogenic amylase;
(c) saccharifying the liquefied material obtained in step (b) with a carbohydrate-source generating enzyme; and
(d) fermenting the saccharified material using a fermenting microorganism.

In a preferred embodiment the starch-containing material is reduced in size, preferably by dry milling. Step (b) may be carried out in accordance with the liquefaction process of the invention. Steps (c) and (d) may be carried out separately or simultaneously (SSF process).

In another aspect the invention relates to a process for producing a fermentation product, such as ethanol, comprising:
(a) reducing the size of starch-containing material
(b) liquefying the product of step (a) with at least one amylase and at least one esterase;
(c) saccharifying the liquefied material obtained in step (b) with a carbohydrate-generating enzyme; and
(d) fermenting the saccharified material using a fermenting microorganism.

In a preferred embodiment the starch-containing material is reduced in size, preferably by dry milling. Step (b) may be carried out in accordance with the liquefaction process of the invention. Steps (c) and (d) may be carried out separately or simultaneously (SSF process).

The invention also provides a process for producing a fermentation product, such as ethanol, comprising
(a) reducing the size of starch-containing material
(b) i) pre-treating a slurry of said starch-containing material with at least one esterase, and
   ii) liquefying the pre-treated slurry with an alpha-amylase;
(c) saccharifying the liquefied material obtained in step (b) with a carbohydrate-generating enzyme; and,
(d) fermenting the saccharified material using a fermenting microorganism.

Steps (b) and (c) may be carried out in accordance with the liquefaction process of the invention. Steps (c) and (d) may be carried out separately or simultaneously (SSF process).

### DESCRIPTION OF THE INVENTION

The present invention provides an improved liquefaction process suitable as a step in processes for producing, e.g., syrups or fermentation products. As a result of the process of the invention formation of retrograded starch is prevented or at least reduced and thus more carbohydrates are liberated from the starch-containing raw starting material.

### Starting materials

The raw starch-containing starting material is reduced in size, preferably by dry milling. Dry milling processes are well-known in the art and generally involve the step of grinding/milling starch-containing material, such as whole cereal grains, in a dry or substantially dry state. However, other techniques is capable of reducing the size of the starch-containing material are also contemplated and within the scope of the invention.

In ethanol production dry milling generally includes the steps of grinding/milling whole cereal grains to produce a meal, and subjecting the meal to liquefaction, saccharification, fermentation and optionally recovery by, e.g., distillation.

The starting material is generally selected based on the desired fermentation product and the process employed. Examples of starting materials suitable for use in a process of the present invention include starch-containing raw materials, such as tubers, roots, whole grains, corns, cobs, wheat, barley, rye, milo or cereals, sugar-containing raw materials, such as molasses, fruit materials, sugar, cane or sugar beet, potatoes, and cellulose-containing materials, such as wood or plant residues. Starch-containing whole corn grains are the preferred raw starting material for the liquefaction and fermentation product, such as ethanol, production processes of the invention.

### Fermentation products

Fermentation products contemplated according to the invention include alcohols (e.g., ethanol, methanol, butanol, 1,3-propanediol); organic acids (e.g., citric acid, acetic acid, itaconic acid, lactic acid, gluconic acid, gluconate, lactic acid, succinic acid, 2,5-diketo-D-gluconic acid); ketones (e.g., acetone); amino acids (e.g., glutamic acid); gases (e.g., H₂ and CO₂), and more complex compounds, including, for example, antibiotics (e.g., penicillin and tetracycline); enzymes; vitamins (e.g., riboflavin, B₁₂, beta-carotene); and hormones.

### Liquefaction

The present inventors have found that liquefaction of starch-containing material may be improved by treating said starch-containing material with at least one alpha-amylase and at least one maltogenic amylase or alternatively with at least one amylase and at least one esterase.

Thus, in the first aspect the invention provides a process for liquefying starch-containing material comprising the step of treating said starch-containing material with at least one alpha-amylase and at least one maltogenic amylase.

According to the second aspect the invention provides a process for liquefying starch-containing material comprising the step of treating starch-containing material with at least one amylase and at least one esterase.

Without being limited to any theory it is believed that the treatment with a combination of amylase and esterase reduces the formation of retrograded starch. The esterase is believed to attack lipids present in, e.g., dry milled starch-containing material, such as corn, to produce smaller molecules that are less likely to produce starch-lipid complexes referred to as retrograded starch which are created during jet cooking. Further, esterases catalyze a reaction between dendrimer and soluble starch chains occurring at the oil inter-phase to form a new architecture that prevents the formation of the lipid-starch complex during jet cooking and liquefaction. It may also reduce the amount of amylase needed to carry out liquefaction.

According to the present invention "liquefaction" is a process in which starch-containing material, preferably (whole) grain raw material, is broken down (hydrolyzed) into maltodextrins (dextrins). According to the invention liquefaction may be carried out by heating the slurry of 20-40 wt-%, preferably 25-35 wt-% starch-containing material and water to between 20-105°C, preferably 60-95°C and adding the enzymes to initiate liquefaction (thinning). The slurry may then be jet-cooked at a temperature between 95-140°C, preferably 105-125°C to complete gelatinization of the slurry. Then the slurry is cooled to 60-95°C and more enzyme(s) is(are) added to finalize hydrolysis (secondary liquefaction).

In one embodiment of the invention the liquefaction is carried out as a multi-stage process, such as a three-stage process, where the first stage is performed at a temperature in the range from 80 to 105°C, the second stage at a temperature in the range between 65 to 95°C, and the third stage at a temperature between 40-75°C.

In a preferred embodiment three stages are carried out at the following temperature stages: a first stage: 80-95°C, a second stage: 75-85°C, and third stage: 60 to 70°C. According to the invention the holding time for the first stage may be from 10 to 90 minutes, 30-120 minutes for the second stage, and 30-120 minutes for the third stage.

A liquefaction process of the invention may typically be carried out at pH 4.5-6.5, in particular at a pH between 5 and 6.

The amylase may be any amylase, preferred an amylase mentioned in the "Amylases"-section below. In a preferred embodiment the amylase is an alpha-amylase and/or a maltogenic amylase. The esterase may be any esterase, preferably an esterase mentioned in the section "Esterases". Preferred esterases are lipases, phospholipases, and cutinases, or mixtures thereof. A liquefaction process of the invention or a pre-treatment step of the invention may be carried out in the presence of a fatty acid oxidizing enzyme, preferably a lipoxygenase, as will be defined further below in the section "Fatty acid oxidizing enzymes".

In one embodiment the liquefaction process comprises the steps of:
i) pre-treating a slurry of starch-containing material with at least one esterase, and
ii) liquefying the pre-treated slurry with an alpha-amylase.

The pre-treated material is preferably reduced in size, preferably by dry milling. As mentioned above the liquefaction may be carried out as a three-stage hot slurry process. In an embodiment of the invention the esterase is used together with a maltogenic amylase during the pre-treatment. In another embodiment an esterase, a maltogenic amylase, and an alpha-amylase are present during pre-treatment. In a further embodiment an esterase, a maltogenic amylase and carbohydrate-source generating enzymes, such as a glucoamylase and optionally a fungal acid alpha-amylase are present during pre-treatment.

In another embodiment of the process of the invention starch-containing material reduced in size, e.g., by dry milling, is liquefied by treatment with an esterase, maltogenic amylase and/or an alpha-amylase without or without pre-treatment.

In a preferred embodiment the pre-treatment is carried out by subjecting an aqueous slurry of preferably starch-containing material reduced in size, e.g., by dry milling, to an esterase, preferably a lipase, a maltogenic amylase and an alpha-amylase, preferably an acid amylase, such as a fungal acid alpha-amylase followed by liquefaction with an alpha-amylase.

The process is preferably carried out in an aqueous hot slurry at a temperature in the range from 20-105°C, preferably 60-95°C.

### Fermentation product process

Fermentation product, such as ethanol, production processes of the invention generally involve the steps of reducing the size of the starch-containing starting material, e.g., by dry milling, liquefaction, saccharification, fermentation and optionally recovery, e.g., by distillation. In production processes for producing a fermentation product, such as ethanol, the raw starch-containing material, such as whole grains, preferably corn, is reduced in size, e.g., by dry milling, in order to open up the structure and allow for further processing.

In one aspect the invention provides a process for producing a fermentation product, such as ethanol, comprising
(a) reducing the size of starch-containing material
(b) liquefying the product of step (a) with at least one alpha-amylase and at least one maltogenic amylase;
(c) saccharifying the liquefied material obtained in step (b) with a carbohydrate-source generating enzyme; and
(d) fermenting the saccharified material using a fermenting microorganism.

Step (b) may be carried out in accordance with the liquefaction process of the invention.

In another aspect the invention provides a process for producing a fermentation product, such as ethanol, comprising
(a) reducing the size of starch-containing material;
(b) liquefying the product of step (a) with at least one amylase and at least one esterase;
(c) saccharifying the liquefied material obtained in step (b) with a carbohydrate-generating enzyme; and
(d) fermenting the saccharified material using a fermenting microorganism.

Step (b) may be carried out in accordance with the liquefaction process of the invention.

In yet another aspect the invention provides a process for producing a fermentation product, such as ethanol, comprising
(a) reducing the size of starch-containing material
(b) i) pre-treating a slurry of said starch-containing material with at least one esterase,
   ii) liquefying the pre-treated slurry with an alpha-amylase.
(c) saccharifying the liquefied material obtained in step (b) with a carbohydrate-generating enzyme; and
(d) fermenting the saccharified material using a fermenting microorganism.

Step (b) is carried out in accordance with the liquefaction process of the invention. Steps (c) and (d) may be carried out sequentially or simultaneously (SSF process).

The fermentation step may be followed by an optional recovery, such as distillation of the fermentation product.

### Saccharification

"Saccharification" is a step in which the maltodextrin (such as, product from the liquefaction process) is converted to low molecular sugars DP₁₋₃ (i.e., carbohydrate source) that can be metabolized by a fermenting microorganism, such as yeast. A saccharification step in a fermentation product producing process of the invention may be carried out using a saccharification step well known in the art. Saccharification is typically performed enzymatically using at least one or more carbohydrate-source generating enzymes, such as a glucoamylase. The saccharification step in a process for producing ethanol of the invention may be a well known saccharification step in the art. In one embodiment glucoamylase, alpha-glucosidases and/or acid alpha-amylase is used for treating the liquefied starch-containing material. A full saccharification step may last up to from about 24 to about 72 hours or more, and is often carried out at temperatures from about 30 to 65°C and at a pH between 4 and 5, normally at about pH 4.5. However, it is often more preferred to do a pre-saccharification step, lasting for about 40 to 90 minutes at temperature of between 30-65°C, typically about 60°C, followed by complete saccharification during fermentation in a simultaneous saccharification and fermentation process (SSF process). In ethanol production saccharification is usually carried out as a simultaneous saccharification and fermentation (SSF) process, in which there is no holding stage for the saccharification, meaning that fermenting microorganism, such as yeast, and enzyme(s) is(are) added together. In SSF processes, it is common to introduce a pre-saccharification step at a temperature, e.g., above 50°C, just prior to the fermentation.

### Fermentation

In ethanol production, the fermenting microorganism is preferably yeast, which is applied to the saccharified mash. "Fermenting microorganism" refers to any organism suitable for use in a desired fermentation process. Suitable fermenting microorganisms are according to the invention able to ferment, i.e., convert, sugars, such as glucose or maltose, directly or indirectly into the desired fermentation product. Examples of fermenting microorganisms include fungal organisms, such as yeast. Preferred yeast includes strains of the *Saccharomyces* spp., and in particular, *Saccharomyces cerevisiae.* Commercially available yeast includes, e.g., RED STAR®/Lesaffre ETHANOL RED (available from Red Star/Lesaffre, USA) FALI (available from Fleischmann's Yeast, a division of Burns Philp Food Inc., USA), SUPERSTART (available from Alltech), GERT STRAND (available from Gert Strand AB, Sweden) and FERMIOL (available from DSM Specialties). In preferred embodiments, yeast is applied to the mash and the fermentation is ongoing for 24-96 hours, such as typically 35-60 hours. In a preferred embodiment the temperature is generally between 26-34°C, in particular about 32°C, and the pH is generally from pH 3-6, preferably around pH 4-5. Yeast cells are preferably applied in amounts of 10⁵ to 10¹², preferably from 10⁷ to 10¹⁰, especially 5x10⁷ viable yeast count per mL of fermentation broth. During the ethanol producing phase the yeast cell count should preferably be in the range from 10⁷ to 10¹⁰, especially around 2 x 10⁸. Further guidance in respect of using yeast for fermentation can be found in, e.g., "The alcohol Textbook" (Editors K. Jacques, T.P. Lyons and D.R.Kelsall, Nottingham University Press, United Kingdom 1999).

### Recovery

Following fermentation, the mash may be recovered by, e.g., distilled, to extract the fermentation product, such as alcohol product (especially ethanol). In the case where the end product is ethanol it may be used as, e.g., fuel ethanol; drinking ethanol, i.e., potable neutral spirits; or industrial ethanol.

### Starch Conversion

The liquefaction process of the invention may also be included in a traditional starch conversion process for producing syrups such as glucose, maltose, malto-oligosaccharides and isomalto-oligosaccharides.

### Amylases

Suitable amylases include alpha-amylases, beta-amylases and maltogenic amylases, or mixtures thereof.

### Alpha-amylases

According to the invention preferred alpha-amylases are of fungal or bacterial origin.

In an embodiment the alpha-amylase is a *Bacillus* alpha-amylase, such as, derived from a strain of *B*. *licheniformis, B. amyloliquefaciens, and B*. *stearothermophilus.* Other alpha-amylases include alpha-amylase derived from a strain of the *Bacillus* sp. NCIB 12289, NCIB 12512, NCIB 12513 or DSM 9375, all of which are described in detail in WO 95/26397, and the alpha-amylase described by Tsukamoto et al., Biochemical and Biophysical Research Communications, 151 (1988), pp. 25-31.

The alpha-amylase may also be a variant and/or hybrid, especially one described in any of WO 96/23873, WO 96/23874, WO 97/41213, WO 99/19467, WO 00/60059, and WO 02/10355 (all documents hereby incorporated by reference). Specifically contemplated alpha-amylase variants are disclosed in US Patent Nos. 6,093,562, 6,297,038 or US Patent No. 6,187,576 (hereby incorporated by reference) and include *Bacillus stearothermophilus* alpha-amylase (BSG alpha-amylase) variants having a deletion of one or two amino acid in positions R179 to G182, preferably a double deletion disclosed in WO 1996/023873 - see e.g., page 20, lines 1-10 (hereby incorporated by reference), preferably corresponding to delta(181-182) compared to the wild-type BSG alpha-amylase amino acid sequence set forth in SEQ ID NO:3 disclosed in WO 99/19467 or deletion of amino acids R179 and G180 using SEQ ID NO:3 in WO 99/19467 for numbering (which reference is hereby incorporated by reference). Even more preferred are *Bacillus* alpha-amylases, especially *Bacillus stearothermophilus* alpha-amylase, which have a double deletion corresponding to delta(181-182) and further comprise a N193F substitution (also denoted I181* + G182* + N193F) compared to the wild-type BSG alpha-amylase amino acid sequence set forth in SEQ ID NO:3 disclosed in WO 99/19467.

A hybrid alpha-amylase specifically contemplated comprises 445 C-terminal amino acid residues of the *Bacillus licheniformis* alpha-amylase (shown in SEQ ID NO: 4 of WO 99/19467) and the 37 N-terminal amino acid residues of the alpha-amylase derived from *Bacillus amyloliquefaciens* (shown in SEQ ID NO: 5 of WO 99/19467), with the following substitution: G48A+T49I+G107A+H156Y+A181T+N190F+I201F+A209V+Q264S (using the numbering in SEQ ID NO: 4 of WO 99/19467). Especially preferred are variants having one or more of the mutations H154Y, A181T, N190F, A209V and Q264S and/or deletion of two residues between positions 176 and 179, preferably deletion of E178 and G179 (using the SEQ ID NO: 5 numbering of WO 99/19467).

Other contemplated bacterial alpha-amylases are KSM-K36 alpha-amylase disclosed in EP 1,022,334 and deposited as FERM BP 6945 and KSM-K38 alpha-amylase disclosed in EP 1,022,334, and deposited as FERM BP-6946. Also variants therefore are contemplated, in particular the variants disclosed in WO 02/31124 (from Novozymes A/S).

Other alpha-amylase includes alpha-amylases derived from a strain of *Aspergillus,* such as, *Aspergillus oryzae* and *Aspergillus niger* alpha-amylases. In a preferred embodiment, the alpha-amylase is an acid alpha-amylase. In a more preferred embodiment the acid alpha-amylase is an acid fungal alpha-amylase or an acid bacterial alpha-amylase. More preferably, the acid alpha-amylase is an acid fungal alpha-amylase derived from the genus *Aspergillus.* A commercially available acid fungal amylase is SP288 (available from Novozymes A/S, Denmark).

In an embodiment the alpha-amylase is an acid alpha-amylase. The term "acid alpha-amylase" means an alpha-amylase (E.C. 3.2.1.1) which added in an effective amount has activity at a pH in the range of 3.0 to 7.0, preferably from 3.5 to 6.0, or more preferably from 4.0-5.0.

A preferred acid fungal alpha-amylase is a Fungamyl-like alpha-amylase. In the present disclosure, the term "Fungamyl-like alpha-amylase" indicates an alpha-amylase which exhibits a high identity, i.e., more than 50%, 55%, 60%, 65%, 70%, 75%, 80%, 85% 90%, 95 or even 99% identical to the amino acid sequence shown in SEQ ID NO: 10 in WO 96/23874. When used as a maltose generating enzyme fungal alpha-amylases may be added in an amount of 0.001-1.0 AFAU/g DS, preferably from 0.002-0.5 AFAU/g DS, preferably 0.02-0.1 AFAU/g DS.

Preferably the alpha-amylase is an acid alpha-amylase, preferably from the genus *Aspergillus,* preferably of the species *Aspergillus niger.* In a preferred embodiment the acid fungal alpha-amylase is the one from *A. niger* disclosed as "AMYA_ASPNG" in the Swiss-prot/TeEMBL database under the primary accession no. P56271. Also variant of set acid fungal amylase having at least 70% identity, such as at least 80% or even at least 90% identity thereto is contemplated.

Other contemplated alpha-amylases include the hybrid alpha-amylases disclosed in WO 2005/003311 (hereby incorporated by reference).

Preferred commercial compositions comprising an alpha-amylase include MYCOLASE™ from DSM; BAN™, TERMAMYL™ SC, FUNGAMYL™, LIQUOZYME™ X and SAN™ SUPER, SAN™ EXTRA L from Novozymes A/S, Denmark) and CLARASE™ L-40,000, DEX-LO™, SPEYME FRED, SPEZYME™ AA, and SPEZYME™ DELTA AA (Genencor Int., USA), and the acid fungal alpha-amylase sold under the trade name SP 288 (available from Novozymes A/S, Denmark).

The alpha-amylase may be added in amounts as are well-known in the art. When measured in AAU units the acid alpha-amylase activity is preferably present in an amount of 5-50,0000 AAU/kg of DS, in an amount of 500-50,000 AAU/kg of DS, or more preferably in an amount of 100-10,000 AAU/kg of DS, such as 500-1,000 AAU/kg DS. Fungal acid alpha-amylase are preferably added in an amount of 10-10,000 AFAU/kg of DS, in an amount of 500-2,500 AFAU/kg of DS, or more preferably in an amount of 100-1,000 AFAU/kg of DS, such as approximately 500 AFAU/kg DS.

### Maltogenic amylases

The amylase may also be a maltogenic alpha-amylase. A "maltogenic alpha-amylase" (glucan 1,4-alpha-maltohydrolase, E.C. 3.2.1.133) is able to hydrolyze amylose and amylopectin to maltose in the alpha-configuration. A maltogenic alpha-amylase from *Bacillus stearothermophilus* strain NCIB 11837 is commercially available from Novozymes A/S under the tradename MALTOGENASE™. Maltogenic alpha-amylases are described in US Patent Nos. 4,598,048, 4,604,355 and 6,162,628, which are hereby incorporated by reference.

### Esterases

As used herein, an "esterase" also referred to as a carboxylic ester hydrolyases, refers to enzymes acting on ester bonds, and includes enzymes classified in EC 3.1.1 Carboxylic Ester Hydrolases according to Enzyme Nomenclature (available at http://www.chem.qmw.ac.uk/iubmb/enzyme or from Enzyme Nomenclature 1992, Academic Press, San Diego, California, with Supplement 1 (1993), Supplement 2 (1994), Supplement 3 (1995), Supplement 4 (1997) and Supplement 5, in Eur. J. Biochem. 1994, 223, 1-5; Eur. J. Biochem. 1995, 232, 1-6; Eur. J. Biochem. 1996, 237, 1-5; Eur. J. Biochem. 1997, 250; 1-6, and Eur. J. Biochem. 1999, 264, 610-650; respectively). Non-limiting examples of esterases include arylesterase, triacylglycerol lipase, acetylesterase, acetylcholinesterase, cholinesterase, tropinesterase, pectinesterase, sterol esterase, chlorophyllase, L-arabinonolactonase, gluconolactonase, uronolactonase, tannase, retinyl-palmitate esterase, hydroxybutyrate-dimer hydrolase, acylglycerol lipase, 3-oxoadipate enol-lactonase, 1,4-lactonase, galactolipase, 4-pyridoxolactonase, acylcarnitine hydrolase, aminoacyl-tRNA hydrolase, D-arabinonolactonase, 6-phosphogluconolactonase, phospholipase A1, 6-acetylglucose deacetylase, lipoprotein lipase, dihydrocoumarin lipase, limonin-D-ring-lactonase, steroid-lactonase, triacetate-lactonase, actinomycin lactonase, orsellinate-depside hydrolase, cephalosporin-C deacetylase, chlorogenate hydrolase, alpha-amino-acid esterase, 4-methyloxaloacetate esterase, carboxymethylenebutenolidase, deoxylimonate A-ring-lactonase, 2-acetyl-1-alkylglycerophosphocholine esterase, fusarinine-C ornithinesterase, sinapine esterase, wax-ester hydrolase, phorbol-diester hydrolase, phosphatidylinositol deacylase, sialate O-acetylesterase, acetoxybutynylbithiophene deacetylase, acetylsalicylate deacetylase, methylumbelliferyl-acetate deacetylase, 2-pyrone-4,6-dicarboxylate lactonase, N-acetylgalactosaminoglycan deacetylase, juvenile-hormone esterase, bis(2-ethylhexyl)phthalate esterase, protein-glutamate methylesterase, 11-cis-retinyl-palmitate hydrolase, all-trans-retinyl-palmitate hydrolase, L-rhamnono-1,4-lactonase, 5-(3,4-diacetoxybut-1-ynyl)-2,2'-bithiophene deacetylase, fatty-acyl-ethyl-ester synthase, xylono-1,4-lactonase, N-acetylglucosaminylphosphatidylinositol deacetylase, cetraxate benzylesterase, acetylalkylglycerol acetylhydrolase, and acetylxylan esterase.

Preferred esterases for use in the present invention are lipolytic enzymes, such as, lipases (as classified by EC 3.1.1.3, EC 3.1.1.23 and/or EC 3.1.1.26) and phospholipases (as classified by EC 3.1.1.4 and/or EC 3.1.1.32, including lysophospholipases as classified by EC 3.1.1.5). Other preferred esterases are cutinases (as classified by EC 3.1.1.74).

Examples of effective amounts of esterase are from 0.01 to 400 LU/g DS (Dry Solids). Preferably, the esterase is used in an amount of 0.1 to 100 LU/g DS, more preferably 0.5 to 50 LU/g DS, and even more preferably 1 to 20 LU/g DS. Further optimization of the amount of esterase can hereafter be obtained using standard procedures known in the art.

In a preferred embodiment the esterase is a lipolytic enzyme, more preferably, a lipase. As used herein, a "lipolytic enzymes" refers to lipases and phospholipases (including lyso-phospholipases). The lipolytic enzyme is preferably of microbial origin, in particular of bacterial, fungal or yeast origin. The lipolytic enzyme used may be derived from any source, including, for example, a strain of *Absidia,* in particular *Absidia* blakesleena and *Absidia* corymbifera, a strain of *Achromobacter,* in particular *Achromobacter iophagus,* a strain of *Aeromonas,* a strain of *Alternaria,* in particular *Alternaria brassiciola,* a strain of *Aspergillus,* in particular *Aspergillus niger* and *Aspergillus flavus,* a strain of *Achromobacter,* in particular *Achromobacter iophagus,* a strain of *Aureobasidium,* in particular *Aureobasidium pullulans,* a strain of *Bacillus,* in particular *Bacillus pumilus, Bacillus strearothermophilus* and *Bacillus subtilis,* a strain of *Beauveria,* a strain of *Brochothrix,* in particular *Brochothrix thermosohata,* a strain of *Candida,* in particular *Candida cylindracea (Candida rugosa), Candida paralipolytica,* and *Candida antarctica,* a strain of *Chromobacter,* in particular *Chromobacter viscosum,* a strain of *Coprinus,* in particular *Coprinus cinerius,* a strain of *Fusarium,* in particular *Fusarium oxysporum, Fusarium solani, Fusarium solani pisi,* and *Fusarium roseum culmorum,* a strain of *Geotricum,* in particular *Geotricum penicillatum,* a strain of *Hansenula,* in particular *Hansenula anomala,* a strain of *Humicola,* in particular *Humicola brevispora, Humicola brevis var. thermoidea,* and *Humicola insolens,* a strain of *Hyphozyma,* a strain of *Lactobacillus,* in particular *Lactobacillus curvatus,* a strain of *Metarhizium,* a strain of *Mucor,* a strain of *Paecilomyces,* a strain of *Penicillium,* in particular *Penicillium cyclopium,* Penicillium crustosum and *Penicillium expansum,* a strain of *Pseudomonas* in particular *Pseudomonas aeruginosa, Pseudomonas alcaligenes, Pseudomonas cepacia* (syn. *Burkholderia cepacia), Pseudomonas fluorescens, Pseudomonas fragi, Pseudomonas maltophilia, Pseudomonas mendocina, Pseudomonas* mephitica lipolytica, *Pseudomonas alcaligenes,* Pseudomonas plantari, *Pseudomonas pseudoalcaligenes, Pseudomonas putida, Pseudomonas stutzeri,* and *Pseudomonas wisconsinensis,* a strain of *Rhizoctonia,* in particular *Rhizoctonia solani,* a strain of *Rhizomucor,* in particular *Rhizomucor miehei,* a strain of *Rhizopus,* in particular *Rhizopus japonicus, Rhizopus microsporus* and *Rhizopus nodosus,* a strain of *Rhodosporidium,* in particular *Rhodosporidium toruloides,* a strain of *Rhodotorula,* in particular *Rhodotorula glutinis,* a strain of *Sporobolomyces,* in particular *Sporobolomyces shibatanus,* a strain of *Thermomyces,* in particular *Thermomyces lanuginosus* (formerly *Humicola lanuginosa*), a strain of *Thiarosporella,* in particular *Thiarosporella phaseolina,* a strain of *Trichoderma,* in particular *Trichoderma harzianum,* and *Trichoderma reesei,* and/or a strain of *Verticillium.*

In a preferred embodiment, the lipolytic enzyme is derived from a strain of *Aspergillus,* a strain of *Achromobacter,* a strain of *Bacillus,* a strain of *Candida,* a strain of *Chromobacter,* a strain of *Fusarium,* a strain of *Humicola,* a strain of *Hyphozyma,* a strain of *Pseudomonas,* a strain of *Rhizomucor,* a strain of *Rhizopus,* or a strain of *Thermomyces.*

In more preferred embodiments, the lipolytic enzyme is a lipase. Lipases may be applied herein for their ability to modify the structure and composition of triglyceride oils and fats in the fermentation media (including fermentation yeast), for example, resulting from a corn substrate. Lipases catalyze different types of triglyceride conversions, such as hydrolysis, esterification and transesterification. Suitable lipases include acidic, neutral and basic lipases, as are well-known in the art, although acidic lipases (such as, e.g., the lipase G AMANO 50, available from Amano) appear to be more effective at lower concentrations of lipase as compared to either neutral or basic lipases. Preferred lipases for use in the present invention included Candida antarcitca lipase and *Candida cylindracea* lipase. More preferred lipases are purified lipases such as *Candida antarcitca* lipase (lipase A), *Candida antarcitca* lipase (lipase B), *Candida cylindracea* lipase, and *Penicillium camembertii* lipase.

The lipase the one disclosed in EP 258,068-A or may be a lipase variant such as a variant disclosed in WO 00/60063 or WO 00/32758 which is hereby incorporated by reference. Preferred commercial lipases include LECITASE™, LIPOLASE™, LIPEX™ and NOVOZYM® 735 (available from Novozymes A/S, Denmark) and G AMANO™ 50 (available from Amano).

Lipases are preferably added in amounts from about 1 to 400 LU/g DS, preferably 1 to 10 LU/g DS, and more preferably 1 to 5 LU/g DS.

In another preferred embodiment of the present invention, the at least one esterase is a cutinase. Cutinases are enzymes which are able to degrade cutin. The cutinase may be derived from any source. In a preferred embodiment, the cutinase is derived from a strain of *Aspergillus,* in particular *Aspergillus oryzae,* a strain of *Alternaria,* in particular *Alternaria brassiciola,* a strain of *Fusarium,* in particular *Fusarium solani, Fusarium solani pisi, Fusarium roseum culmorum,* or *Fusarium roseum sambucium,* a strain of *Helminthosporum,* in particular *Helminthosporum sativum,* a strain of *Humicola,* in particular *Humicola insolens,* a strain of *Pseudomonas,* in particular *Pseudomonas mendocina,* or *Pseudomonas putida,* a strain of *Rhizoctonia,* in particular *Rhizoctonia solani,* a strain of *Streptomyces,* in particular *Streptomyces scabies,* or a strain of *Ulocladium,* in particular *Ulocladium consortiale.* In a most preferred embodiment the cutinase is derived from a strain of *Humicola insolens,* in particular the strain *Humicola insolens* DSM 1800. *Humicola insolens* cutinase is described in WO 96/13580 which is herby incorporated by reference. The cutinase may be a variant such as one of the variants disclosed in WO 00/34450 and WO 01/92502 which is hereby incorporated by reference. Preferred cutinase variants include variants listed in Example 2 of WO 01/92502 which are hereby specifically incorporated by reference. An effective amount of cutinase is between 0.01 and 400 LU/g DS, preferably from about 0.1 to 100 LU/g DS, more preferably, 1 to 50 LU/g DS. Further optimization of the amount of cutinase can hereafter be obtained using standard procedures known in the art.

In another preferred embodiment, the at least one esterase is a phospholipase. As used herein, the term phospholipase is an enzyme which has activity towards phospholipids. Phospholipids, such as lecithin or phosphatidylcholine, consist of glycerol esterified with two fatty acids in an outer (sn-1) and the middle (sn-2) positions and esterified with phosphoric acid in the third position; the phosphoric acid, in turn, may be esterified to an amino-alcohol. Phospholipases are enzymes which participate in the hydrolysis of phospholipids. Several types of phospholipase activity can be distinguished, including phospholipases A₁ and A₂ which hydrolyze one fatty acyl group (in the sn-1 and sn-2 position, respectively) to form lysophospholipid; and lysophospholipase (or phospholipase B) which can hydrolyze the remaining fatty acyl group in lysophospholipid. Phospholipase C and phospholipase D (phosphodiesterases) release diacyl glycerol or phosphatidic acid respectively.

The term phospholipase includes enzymes with phospholipase activity, e.g., phospholipase A (A₁ or A₂), phospholipase B activity, phospholipase C activity or phospholipase D activity. The term "phospholipase A" used herein in connection with an enzyme of the invention is intended to cover an enzyme with Phospholipase A₁ and/or Phospholipase A₂ activity. The phospholipase activity may be provided by enzymes having other activities as well, such as, e.g., a lipase with phospholipase activity. The phospholipase activity may, e.g., be from a lipase with phospholipase side activity. In other embodiments of the invention the phospholipase enzyme activity is provided by an enzyme having essentially only phospholipase activity and wherein the phospholipase enzyme activity is not a side activity.

The phospholipase may be of any origin, e.g., of animal origin (such as, e.g., mammalian), e.g., from pancreas (e.g., bovine or porcine pancreas), or snake venom or bee venom. Alternatively, the phospholipase may be of microbial origin, e.g., from filamentous fungi, yeast or bacteria, such as the genus or species *Aspergillus,* e.g., *A. niger Dictyostelium,* e.g., *D. discoideum; Mucor,* e.g., *M. javanicus, M. mucedo, M. subtilissimus; Neurospora,* e.g., *N. crassa; Rhizomucor,* e.g., *R. pusillus; Rhizopus,* e.g., *R. arrhizus, R. japonicus, R. stolonifer; Sclerotinia,* e.g., S. *libertiana; Trichophyton,* e.g., T. rubrum; Whetzelinia, e.g., W. sclerotiorum; Bacillus, e.g., *B*. *megaterium, B. subtilis; Citrobacter,* e.g., *C. freundii*; *Enterobacter,* e.g., *E*. *aerogenes, E. cloacae, Edwardsiella, E*. *tarda; Erwinia,* e.g., *E. herbicola*; *Escherichia,* e.g., *E*. *coli*; *Klebsiella,* e.g., *K. pneumoniae*; *Proteus,* e.g., P. *vulgaris*; *Providencia,* e.g., *P. stuartii*; *Salmonella,* e.g., S. typhimurium; Serratia, e.g., S. *liquefasciens, S. marcescens*; *Shigella,* e.g., S. *flexneri*; *Streptomyces,* e.g., S. *violeceoruber,* Yersinia, e.g., Y. *enterocolitica.* Thus, the phospholipase may be fungal, e.g., from the class *Pyrenomycetes,* such as the genus *Fusarium,* such as a strain of *F*. *culmorum, F. heterosporum, F. solani,* or a strain of *F. oxysporum.* The phospholipase may also be from a filamentous fungus strain within the genus *Aspergillus,* such as a strain of *Aspergillus awamori, Aspergillus foetidus, Aspergillus japonicus, Aspergillus niger* or *Aspergillus oryzae.* Preferred commercial phospholipases include LECITASE™ and LECITASE™ ULTRA (available from Novozymes A/S, Denmark).

An effective amount of phosphorlipase is between 0.01 and 400 LU/g DS, preferably from about 0.1 to 100 LU/g DS, more preferably, 1 to 50 LU/g DS. Further optimization of the amount of phosphorlipase can hereafter be obtained using standard procedures known in the art.

### Fatty acid oxidizing enzymes

The term "fatty acid oxidizing enzyme" means at least one of such enzymes.

In the present context, a "fatty acid oxidizing enzyme" is an enzyme which hydrolyzes the substrate linoleic acid more efficiently than the substrate syringaldazine. "More efficiently" means with a higher reaction rate. This can be tested using the method described in Example 2 of WO 03/035972 (hereby incorporated by reference), and calculating the difference between (1) absorbancy increase per minute on the substrate linoleic acid (absorbancy at 234 nm), and (2) absorbancy increase per minute on the substrate syringaldazine (absorbancy at 530 nm), i.e., by calculating the Reaction Rate Difference (RRD) = (d(A₂₃₄)/dt - d(A₅₃₀)/dt). If the RRD is above zero, the enzyme in question qualifies as a fatty acid oxidizing enzyme as defined herein. If the RRD is zero, or below zero the enzyme in question is not a fatty acid oxidizing enzyme.

In particular embodiments, the RRD is at least 0.05, 0.10, 0.15, 0.20, or at least 0.25 absorbancy units/minute.

In a particular embodiment the enzymes are well-defined. Still further, for the method of Example 2 of WO 03/035972 the enzyme dosage is adjusted so as to obtain a maximum absorbancy increase per minute at 234 nm, or at 530 nm. In particular embodiments, the maximum absorbancy increase is within the range of 0.05-0.50; 0.07-0.4; 0.08-0.3; 0.09-0.2; or 0.10-0.25 absorbancy units pr. min. The enzyme dosage may for example be in the range of 0.01-20; 0.05-15; or 0.10-10 mg enzyme protein per ml.

In the alternative, a "fatty acid oxidizing enzyme" may be defined as an enzyme capable of oxidizing unsaturated fatty acids more efficiently than syringaldazine. The activity of the enzyme could be compared in a standard oximeter setup as described in Example 1 of the present application at pH 6 and 30°C including either syringaldazine or linoleic acid as substrates.

In a particular embodiment, the fatty acid oxidizing enzyme is defined as an enzyme classified as EC 1.11.1.3, or as EC 1.13.11.-. EC 1.13.11.- means any of the sub-classes thereof, presently forty-nine: EC 1.13.11.1-EC 1.13.11.49. EC 1.11.1.3 is designated fatty acid peroxidase, and EC 1.13.11.- is designated oxygenases acting on single donors with incorporation of two atoms of oxygen.

In a further particular embodiment, the EC 1.13.11.- enzyme is classified as EC 1.13.11.12, EC 1.13.11.31, EC 1.13.11.33, EC 1.13.11.34, EC 1.13.11.40, EC 1.13.11.44 or EC 1.13.11.45, designated lipoxygenase, arachidonate 12-lipoxygenase, arachidonate 15-lipoxygenase, arachidonate 5-lipoxygenase, arachidonate 8-lipoxygenase, linoleate diol synthase, and linoleate 11-lipoxygenase, respectively).

Examples of effective amounts of fatty acid oxidizing enzyme are from 0.001 to 400 U/g DS (Dry Solids). Preferably, the fatty acid oxidizing enzyme is used in an amount of 0.01 to 100 U/g DS, more preferably 0.05 to 50 U/g DS, and even more preferably 0.1 to 20 U/g DS. Further optimization of the amount of fatty acid oxidizing enzyme can hereafter be obtained using standard procedures known in the art.

### Lipoxygenase

In a preferred embodiment, the fatty acid oxidizing enzyme is a lipoxygenase (LOX), classified as EC 1.13.11.12, which is an enzyme that catalyzes the oxygenation of polyunsaturated fatty acids, especially *cis*,*cis*-1,4-dienes, e.g., linoleic acid and produces a hydroperoxide. But also other substrates may be oxidized, e.g., monounsaturated fatty acids.

Microbial lipoxygenases can be derived from, e.g., *Saccharomyces cerevisiae, Thermoactinomyces vulgaris, Fusarium oxysporum, Fusarium proliferatum, Thermomyces lanuginosus, Pyricularia oryzae,* and strains of *Geotrichum.* The preparation of a lipoxygenase derived from *Gaeumannomyces graminis* is described in Examples 3-4 of WO 02/20730. The expression in *Aspergillus oryzae* of a lipoxygenase derived from *Magnaporthe salvinii* is described in Example 2 of WO 02/086114, and this enzyme can be purified using standard methods, e.g., as described in Example 4 of WO 02/20730.

Lipoxygenase (LOX) may also be extracted from plant seeds, such as soybean, pea, chickpea, and kidney bean. Alternatively, lipoxygenase may be obtained from mammalian cells, e.g., rabbit reticulocytes.

Lipoxygenase activity may be determined as described in the "Materials and Methods" section.

Examples of effective amounts of lipoxygenase (LOX) are from 0.001 to 400 U/g DS (Dry Solids). Preferably, the lipoxygenase is used in an amount of 0.01 to 100 U/g DS, more preferably 0.05 to 50 U/g DS, and even more preferably 0.1 to 20 U/g DS. Further optimization of the amount of lipoxygenase can hereafter be obtained using standard procedures known in the art.

### Carbohydrate-Source Generating Enzyme

The term "carbohydrate-source generating enzyme" includes glucoamylases (being glucose generators), beta-amylases and maltogenic amylases (being maltose generators). A carbohydrate-source generating enzyme is capable of providing energy to the fermenting microorganism(s) used in a process of the invention for producing ethanol and/or may be converting directly or indirectly to a desired fermentation product, preferably ethanol. The carbohydrate-source generating enzyme may be mixtures of enzymes falling within the definition. Especially contemplated mixtures are mixtures of at least a glucoamylase and an alpha-amylase, especially an acid amylase, even more preferred an acid fungal alpha-amylase. The ratio between acidic fungal alpha-amylase activity (AFAU) per glucoamylase activity (AGU) (AFAU per AGU) may in an embodiment of the invention be at least 0.1, in particular at least 0.16, such as in the range from 0.12 to 0.50.

Examples of contemplated glucoamylases, alpha-amylases and beta-amylases are set forth in the sections below.

It is to be understood that the enzymes used according to the invention should be added in effective amounts.

### Glucoamylases

A glucoamylase used according to the invention may be derived from any suitable source, e.g., derived from a microorganism or a plant. Preferred glucoamylases are of fungal or bacterial origin, selected from the group consisting of *Aspergillus* glucoamylases, in particular *A. niger* G1 or G2 glucoamylase (Boel et al. (1984), EMBO J. 3 (5), p. 1097-1102), or variants thereof, such as disclosed in WO 92/00381, WO 00/04136 add WO 01/04273 (from Novozymes, Denmark); the *A. awamori* glucoamylase (WO 84/02921), A. *oryzae* (Agric. Biol. Chem. (1991), 55 (4), p. 941-949), or variants or fragments thereof.

Other *Aspergillus* glucoamylase variants include variants to enhance the thermal stability: G137A and G139A (Chen et al. (1996), Prot. Eng. 9, 499-505); D257E and D293E/Q (Chen et al. (1995), Prot. Engng. 8, 575-582); N182 (Chen et al. (1994), Biochem. J. 301, 275-281); disulphide bonds, A246C (Fierobe et al. (1996), Biochemistry, 35, 8698-8704; and introduction of Pro residues in position A435 and S436 (Li et al. (1997), Protein Engng. 10, 1199-1204. Other glucoamylases include *Athelia rolfsii* (previously denoted *Corticium rolfsii*) glucoamylase (see US Patent No. 4,727,026 and (Nagasaka,Y. et al. (1998) Purification and properties of the raw-starch-degrading glucoamylases from Corticium rolfsii, Appl Microbiol Biotechnol 50:323-330), *Talaromyces* glucoamylases, in particular, derived from *Talaromyces emersonii* (WO 99/28448), *Talaromyces leycettanus* (US Patent No. Re. 32,153), *Talaromyces duponti, Talaromyces thermophilus* (US Patent No. 4,587,215). Bacterial glucoamylases contemplated include glucoamylases from the genus *Clostridium,* in particular C. *thermoamylolyticum* (EP 135,138), and C. *thermohydrosulfuricum* (WO 86/01831).

Commercially available compositions comprising glucoamylase include AMG 200L; AMG 300 L; SAN™ SUPER, SAN™ EXTRA L, SPIRIZYME™ PLUS, SPIRIZYME™ FUEL, SPIRIZYME™ B4U and AMG™ E (from Novozymes A/S); OPTIDEX™ 300 (from Genencor Int.); AMIGASE™ and AMIGASE™ PLUS (from DSM); G-ZYME™ G900, G-ZYME™ and G990 ZR (from Genencor Int.).

Glucoamylases may in an embodiment be added in an amount of 0.02-20 AGU/g DS, preferably 0.1-10 AGU/g DS, such as 2 AGU/g DS.

### Beta-amylases

At least according to the invention the a beta-amylase (E.C 3.2.1.2) is the name traditionally given to exo-acting maltogenic amylases, which catalyze the hydrolysis of 1,4-alpha-glucosidic linkages in amylose, amylopectin and related glucose polymers. Maltose units are successively removed from the non-reducing chain ends in a step-wise manner until the molecule is degraded or, in the case of amylopectin, until a branch point is reached. The maltose released has the beta anomeric configuration, hence the name beta-amylase.

Beta-amylases have been isolated from various plants and microorganisms (W.M. Fogarty and C.T. Kelly, Progress in Industrial Microbiology, vol. 15, pp. 112-115, 1979). These beta-amylases are characterized by having optimum temperatures in the range from 40°C to 65°C and optimum pH in the range from 4.5 to 7. A commercially available beta-amylase from barley is NOVOZYM™ WBA from Novozymes A/S, Denmark and SPEZYME™ BBA 1500 from Genencor Int., USA.

### Production of Enzymes

The enzymes referenced herein may be derived or obtained from any suitable origin, including, bacterial, fungal, yeast or mammalian origin. The term "derived" means in this context that the enzyme may have been isolated from an organism where it is present natively, i.e., the identity of the amino acid sequence of the enzyme are identical to a native enzyme. The term "derived" also means that the enzymes may have been produced recombinantly in a host organism, the recombinant produced enzyme having either an identity identical to a native enzyme or having a modified amino acid sequence, e.g., having one or more amino acids which are deleted, inserted and/or substituted, i.e., a recombinantly produced enzyme which is a mutant and/or a fragment of a native amino acid sequence or an enzyme produced by nucleic acid shuffling processes known in the art. Within the meaning of a native enzyme are included natural variants. Furthermore, the term "derived" includes enzymes produced synthetically by, e.g., peptide synthesis. The term "derived" also encompasses enzymes which have been modified e.g., by glycosylation, phosphorylation, or by other chemical modification, whether in vivo or in vitro. The term "obtained" in this context means that the enzyme has an amino acid sequence identical to a native enzyme. The term encompasses an enzyme that has been isolated from an organism where it is present natively, or one in which it has been expressed recombinantly in the same type of organism or another, or enzymes produced synthetically by, e.g., peptide synthesis. With respect to recombinantly produced enzymes the terms "obtained" and "derived" refers to the identity of the enzyme and not the identity of the host organism in which it is produced recombinantly.

The enzymes may also be purified. The term "purified" as used herein covers enzymes free from other components from the organism from which it is derived. The term "purified" also covers enzymes free from components from the native organism from which it is obtained. The enzymes may be purified, with only minor amounts of other proteins being present. The expression "other proteins" relate in particular to other enzymes. The term "purified" as used herein also refers to removal of other components, particularly other proteins and most particularly other enzymes present in the cell of origin of the enzyme of the invention. The enzyme may be "substantially pure," that is, free from other components from the organism in which it is produced, that is, for example, a host organism for recombinantly produced enzymes. In preferred embodiment, the enzymes are at least 75% (w/w) pure, more preferably at least 80%, at least 85%, at least 90%, at least 95%, at least 96%, at least 97%, at least 98%, or at least 99% pure. In another preferred embodiment, the enzyme is 100% pure.

The enzymes used according to the present invention may be in any form suitable for use in the processes described herein, such as, e.g., in the form of a dry powder or granulate, a non-dusting granulate, a liquid, a stabilized liquid, or a protected enzyme. Granulates may be produced, e.g., as disclosed in US Patent Nos. 4,106,991 and 4,661,452, and may optionally be coated by process known in the art. Liquid enzyme preparations may, for instance, be stabilized by adding stabilizers such as a sugar, a sugar alcohol or another polyol, lactic acid or another organic acid according to established process. Protected enzymes may be prepared according to the process disclosed in EP 238,216.

Even if not specifically mentioned in context of a process of the invention, it is to be understood that the enzyme(s) or agent(s) is(are) used in an "effective amount".

### MATERIALS AND METHODS

### Enzymes:

Alpha-amylase A: *Bacillus stearothermophilus* alpha-amylase variant with the following mutations: I181*+G182*+N193F disclosed in US Patent No. 6,187,576 and available on request from Novozymes A/S, Denmark.

Maltogenic amylase A: Maltogenic amylase derived from *Bacillus stearothermophilus* strain NCIB 11837 disclosed in US Patent No. 4,598,048 and available on request from Novozymes A/S, Denmark.

### Stock solution for iodine method:

0.1 N I₂
dissolve 1.3 g I₂ and 2.0 g KI into 100 mL DI water

### Methods:

### Determination of Homology (Identity)

The term polypeptide "homology" means the degree of identity between two amino acid sequences. The homology may suitably be determined by computer programs known in the art, such as, GAP provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), Journal of Molecular Biology, 48, 443-453. The following settings for polypeptide sequence comparison are used: GAP creation penalty of 3.0 and GAP extension penalty of 0.1.

### Alpha-amylase activity (KNU)

The amylolytic activity may be determined using potato starch as substrate. This method is based on the break-down of modified potato starch by the enzyme, and the reaction is followed by mixing samples of the starch/enzyme solution with an iodine solution. Initially, a blackish-blue color is formed, but during the break-down of the starch the blue color gets weaker and gradually turns into a reddish-brown, which is compared to a colored glass standard.

One Kilo Novo alpha amylase Unit (KNU) is defined as the amount of enzyme which, under standard conditions (i.e., at 37°C +/- 0.05; 0.0003 M Ca²⁺; and pH 5.6) dextrinizes 5260 mg starch dry substance Merck Amylum solubile.

A folder EB-SM-0009.02/01 describing this analytical method in more detail is available upon request to Novozymes A/S, Denmark, which folder is hereby included by reference.

### Determination of FAU activity

One Fungal Alpha-Amylase Unit (FAU) is defined as the amount of enzyme, which breaks down 5.26 g starch (Merck Amylum solubile Erg. B.6, Batch 9947275) per hour based upon the following standard conditions:

| | |
|---|---|
| Substrate: | Soluble starch |
| Temperature | 37°C |
| pH | 4.7 |
| Reaction time | 7-20 minutes |

### Determination of acid alpha-amylase activity (AFAU)

Acid alpha-amylase activity is measured in AFAU (Acid Fungal Alpha-amylase Units), which are determined relative to an enzyme standard.

The standard used is AMG 300 L (from Novozymes A/S, Denmark, glucoamylase wild-type *Aspergillus niger* G1, also disclosed in Boel et al. (1984), EMBO J. 3 (5), p. 1097-1102) and WO 92/00381). The neutral alpha-amylase in this AMG falls after storage at room temperature for 3 weeks from approx. 1 FAU/mL to below 0.05 FAU/mL.

The acid alpha-amylase activity in this AMG standard is determined in accordance with the following description. In this method, 1 AFAU is defined as the amount of enzyme, which degrades 5.260 mg starch dry matter per hour under standard conditions.

Iodine forms a blue complex with starch but not with its degradation products. The intensity of color is therefore directly proportional to the concentration of starch. Amylase activity is determined using reverse colorimetry as a reduction in the concentration of starch under specified analytic conditions.

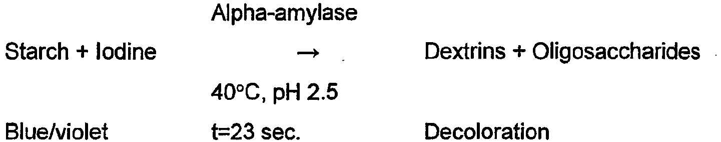

**Standard conditions/reaction conditions: (per minute)**

| | |
|---|---|
| Substrate: | Starch, approx. 0.17 g/L |
| Buffer | Citate, approx. 0.03 M |
| Iodine (I₂): | 0.03 g/L |
| CaCl₂: | 1.85 mM |
| pH: | 2.50 ± 0.05 |
| Incubation temperature: | 40°C |
| Reaction time: | 23 seconds |
| Wavelength: | lambda=590nm |
| Enzyme concentration: | 0.025 AFAU/mL |
| Enzyme working range: | 0.01-0.04 AFAU/mL |

If further details are preferred these can be found in EB-SM-0259.02/01 available on request from Novozymes A/S, Denmark, and incorporated by reference.

### Acid Alpha-amylase Units (AAU)

The acid alpha-amylase activity can be measured in AAU (Acid Alpha-amylase Units), which is an absolute method. One Acid Amylase Unit (AAU) is the quantity of enzyme converting 1 g of starch (100% of dry matter) per hour under standardized conditions into a product having a transmission at 620 nm after reaction with an iodine solution of known strength equal to the one of a color reference.

**Standard conditions/reaction conditions:**

| | |
|---|---|
| Substrate: | Soluble starch. Concentration approx. 20 g DS/L. |
| Buffer: | Citrate, approx. 0.13 M, pH=4.2 |
| Iodine solution: | 40.176 g potassium iodide + 0.088 g iodine/L |
| City water | 15°-20°dH (German degree hardness) |
| pH: | 4.2 |
| Incubation temperature: | 30°C |
| Reaction time: | 11 minutes |
| Wavelength: | 620 nm |
| Enzyme concentration: | 0.13-0.19 AAU/mL |
| Enzyme working range: | 0.13-0.19 AAU/mL |

The starch should be Lintner starch, which is a thin-boiling starch used in the laboratory as colorimetric indicator. Lintner starch is obtained by dilute hydrochloric acid treatment of native starch so that it retains the ability to color blue with iodine. Further details can be found in EP 0140410B2, which disclosure is hereby included by reference.

### Glucoamylase activity (AGI)

Glucoamylase (equivalent to amyloglucosidase) converts starch into glucose. The amount of glucose is determined here by the glucose oxidase method for the activity determination. The method described in the section 76-11 Starch-Glucoamylase Method with Subsequent Measurement of Glucose with Glucose Oxidase in "Approved methods of the American Association of Cereal Chemists". Vol.1-2 AACC, from American Association of Cereal Chemists, (2000); ISBN: 1-891127-12-8.

One glucoamylase unit (AGI) is the quantity of enzyme which will form 1 micromol of glucose per minute under the standard conditions of the method.

**Standard conditions/reaction conditions:**

| | |
|---|---|
| Substrate: | Soluble starch. |
| | Concentration approx. 16 g dry matter/L. |
| Buffer: | Acetate, approx. 0.04 M, pH=4.3 |
| pH: | 4.3 |
| Incubation temperature: | 60°C |
| Reaction time: | 15 minutes |
| Termination of the reaction: | NaOH to a concentration of approximately 0.2 g/L (pH∼9) |
| Enzyme concentration: | 0.15-0.55 AAU/mL |

The starch should be Lintner starch, which is a thin-boiling starch used in the laboratory as colorimetric indicator. Lintner starch is obtained by dilute hydrochloric acid treatment of native starch so that it retains the ability to color blue with iodine.

### Glucoamylase activity (AGU)

The Novo Glucoamylase Unit (AGU) is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes.

An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

| AMG incubation: | |
|---|---|
| Substrate: | maltose 23.2 mM |
| Buffer: | acetate 0.1 M |
| pH: | 4.30 ± 0.05 |
| Incubation temperature: | 37°C ± 1 |
| Reaction time: | 5 minutes |
| Enzyme working range: | 0.5-4.0 AGU/mL |
| Color reaction: | |
| GlucDH: | 430 U/L |
| Mutarotase: | 9 U/L |
| NAD: | 0.21 mM |
| Buffer: | phosphate 0.12 M; 0.15 M NaCl |
| pH: | 7.60 ± 0.05 |
| Incubation temperature: | 37°C ± 1 |
| Reaction time: | 5 minutes |
| Wavelength: | 340 nm |

A folder (EB-SM-0131.02/01) describing this analytical method in more detail is available on request from Novozymes A/S, Denmark.

### Cutinase activity (LU)

The cutinase activity is determined as lipolytic activity determined using tributyrine as substrate. This method was based on the hydrolysis of tributyrin by the enzyme, and the alkali consumption is registered as a function of time. One Lipase Unit (LU) is defined as the amount of enzyme which, under standard conditions (i.e., at 30°C; pH 7.0; with Gum Arabic as emulsifier and tributyrine as substrate) liberates 1 micro mol titrable butyric acid per minute. A folder AF 95/5 describing this analytical method in more detail is available upon request from Novozymes A/S, Denmark, which folder is hereby included by reference.

### Lipoxygenase activity

Lipoxygenase activity is determined spectrophotometrically at 25°C by monitoring the formation of hydroperoxides. For the standard analysis, 10 micro liters enzyme is added to a 1 ml quartz cuvette containing 980 micro liter 25 mM sodium phosphate buffer (pH 7.0) and 10 micro liters of substrate solution (10 mM linoleic acid dispersed with 0.2%(v/v) Tween20 (should not be kept for extended time periods)). The enzyme is typically diluted sufficiently to ensure a turn-over of maximally 10% of the added substrate within the first minute. The absorbance at 234 nm is followed and the rate is estimated from the linear part of the curve. The *cis*-*trans-*conjugated hydro(pero)xy fatty acids are assumed to have a molecular extinction coefficient of 23,000 M⁻¹cm⁻¹.

### Standard iodine method

boil small aliquot (10-20 mLs) of liquefied material in a test tube for several minutes cool in ice bath
add 10-12 drops of the iodine solution
mix and let sample sit in ice water for about 10 minutes

### EXAMPLES

### Example 1

### Liquefaction with an alpha-amylase and a maltogenic amylase

Alpha-amylase A and maltogenic amylase A were added to a slurry of dry milled ground corn (30% solids), heated to 85°C and held for 2 hours. The physical effects from this system were compared to the liquefaction done with only alpha-amylase A.

The mash made with alpha-amylase A and maltogenic amylase A showed less retrograded starch using the standard iodine method as well as being less viscous.

## Claims

1. A process for liquefying starch-containing material comprising treating said starch-containing material with at least one alpha-amylase and a maltogenic amylase (E.C. 3.2.1.133).

2. The process of claim 1, wherein the liquefaction is carried out in multi-stages, such as three stages, preferably a first stage at a temperature in the range from 80 to 105°C, a second stage at a temperature in the range between 65 to 95°C, and a third stage at a temperature between 40-75°C.

3. The process of claim 2, wherein the multi-stage liquefaction is carried out at the following temperature stages: a first stage: 80-95°C, a second stage: 75-85°C, and third stage: 60 to 70°C.

4. The process of claims 2 or 3, wherein the holding time for stage one is 10 to 90 minutes, 30-120 minutes for the second stage and 30-120 minutes for the third stage.

5. The process of any of claims 1-4, wherein the starch-containing material is treated with an esterase and a maltogenic amylase and an alpha-amylase.

6. The process of any of claims 1-5, wherein the starch-containing material is whole grains, preferably corn, wheat, barley, or milo.

7. The process of any of claims 1-6, wherein the amylase or maltogenic amylase is of bacterial origin, preferably a strain of the genus *Bacilllus,* especially *Bacillus stearothermophilus.*

8. The process of any of claims 5-7, wherein the esterase is a lipase, phospholipase, or a cutinase, or a combination thereof.

9. The process of any of claims 1-8, further wherein liquefaction is carried out in the presence of a fatty acid oxidizing enzyme, preferably a lipoxygenase.

10. A process for producing a fermentation product, comprising
(a) reducing the size of starch-containing material;
(b) liquefying the product of step (a) with at least one alpha-amylase and at least one maltogenic amylase as defined in any of claims 1-9;
(c) saccharifying the liquefied material obtained in step (b) with a carbohydrate-source generating enzyme; and
(d) fermenting the saccharified material using a fermenting microorganism.

11. The process of claim 10, wherein steps c) and d) are carried out as a simultaneous saccharification and fermentation step (SSF).

12. The process of any of claims 10 or 11, wherein the carbohydrate-source generating enzyme is a glucoamylase or an alpha-amylase of mixtures thereof, preferably in mixture of acidic fungal alpha-amylase activity (AFAU) per glucoamylase activity (AGU) (AFAU per AGU) of at least 0.1, in particular at least 0.16, such as in the range from 0.12 to 0.50.

13. The process of any of claims 10-12, further comprising distilling the fermented material.

14. The process of any of claims 10-13, wherein said fermenting microorganism is yeast.

15. The process of any of claims 10-14, wherein the fermentation product is ethanol.

## Patentansprüche

1. Verfahren zum Verflüssigen von Stärke enthaltendem Material, umfassend das Behandeln des Stärke enthaltenden Materials mit mindestens einer alpha-Amylase und einer maltogenen Amylase (E.C. 3.2.1.133).

2. Verfahren nach Anspruch 1, wobei die Verflüssigung mehrstufig durchgeführt wird, wie drei Stufen, vorzugsweise eine erste Stufe bei einer Temperatur im Bereich von 80 bis 105°C, einer zweiten Stufe bei einer Temperatur im Bereich zwischen 65 bis 95°C, und einer dritten Stufe bei einer Temperatur zwischen 40-75°C.

3. Verfahren nach Anspruch 2, wobei die mehrstufige Verflüssigung bei den folgenden Temperaturstufen durchgeführt wird: eine erste Stufe: 80-95°C, eine zweite Stufe: 75-85°C, und dritte Stufe: 60 bis 70°C.

4. Verfahren nach Ansprüchen 2 oder 3, wobei die Haltezeit für Stufe eins 10 bis 90 Minuten beträgt, 30-120 Minuten für die zweite Stufe und 30-120 Minuten für die dritte Stufe.

5. Verfahren nach einem beliebigen der Ansprüche 1-4, wobei das Stärke enthaltende Material mit einer Esterase und einer maltogenen Amylase und einer alpha-Amylase behandelt wird.

6. Verfahren nach einem beliebigen der Ansprüche 1-5, wobei das Stärke enthaltende Material Vollkorn, vorzugsweise Mais, Weizen, Gerste oder Hirse ist.

7. Verfahren nach einem beliebigen der Ansprüche 1-6, wobei die Amylase oder maltogene Amylase von bakteriellem Ursprung, vorzugsweise einem Stamm der Gattung *Bacillus,* insbesondere *Bacillus stearothermophilus,* ist.

8. Verfahren nach einem beliebigen der Ansprüche 5-7, wobei die Esterase eine Lipase, Phospholipase oder eine Cutinase, oder eine Kombination davon, ist.

9. Verfahren nach einem beliebigen der Ansprüche 1-8, des Weiteren wobei Verflüssigung in der Gegenwart eines Fettsäure oxidierenden Enzyms durchgeführt wird, vorzugsweise einer Lipoxygenase.

10. Verfahren zum Herstellen eines Fermentationsprodukts, umfassend
(a) Reduzieren der Größe von Stärke enthaltendem Material;
(b) Verflüssigen des Produkts von Schritt (a) mit mindestens einer alpha-Amylase und mindestens einer maltogenen Amylase wie in einem beliebigen der Ansprüche 1-9 definiert;
(c) Verzuckern des in Schritt (b) erhaltenen verflüssigten Materials mit einem Kohlenhydratquelle erzeugenden Enzym; und
(d) Fermentieren des verzuckerten Materials unter Verwendung eines fermentierenden Mikroorganismus.

11. Verfahren nach Anspruch 10, wobei Schritte c) und d) als ein simultaner Verzuckerungs- und Fermentationsschritt *(simultaneous saccharification and fermentation step;* SSF) durchgeführt wird.

12. Verfahren nach einem beliebigen der Ansprüche 10 oder 11, wobei das Kohlenhydratquelle erzeugende Enzym eine Glucoamylase oder eine alpha-Amylase oder Mischungen davon ist, vorzugsweise in Mischung von saurer Pilz-alpha-Amylase Aktivität *(acidic fungal alpha-amylase activity*; AFAU) pro Glucoamylaseaktivität (AGU) (AFAU pro AGU) von mindestens 0,1, insbesondere mindestens 0,16, wie im Bereich von 0,12 bis 0,50.

13. Verfahren nach einem beliebigen der Ansprüche 10-12, des Weiteren umfassend das Destillieren des fermentierten Materials.

14. Verfahren nach einem beliebigen der Ansprüche 10-13, wobei der fermentierende Mikroorganismus Hefe ist.

15. Verfahren nach einem beliebigen der Ansprüche 10-14, wobei das Fermentationsprodukt Ethanol ist.

## Revendications

1. Procédé pour liquéfier une matière contenant de l'amidon comprenant le traitement de ladite matière contenant de l'amidon avec au moins une alpha-amylase et une amylase maltogénique (E.C. 3.2.1.133).

2. Procédé selon la revendication 1, dans lequel la liquéfaction est réalisée en plusieurs étapes, tel que trois étapes, de préférence une première étape à une température allant de 80 à 105°C, une seconde étape à une température allant de 65 à 95°C, et une troisième étape à une température entre 40-75°C.

3. Procédé selon la revendication 2, dans lequel la liquéfaction en plusieurs étapes est réalisée aux températures d'étapes suivantes : une première étape : 80-95°C, une seconde étape : 75-85°C, et une troisième étape : 60 à 70°C.

4. Procédé selon les revendications 2 ou 3, dans lequel le temps de maintien de l'étape une est de 10 à 90 minutes, 30-120 minutes pour la seconde étape et 30-120 minutes pour la troisième étape.

5. Procédé selon l'une quelconque des revendications 1 à 4, dans lequel la matière contenant de l'amidon est traitée avec une estérase et une amylase maltogénique et une alpha-amylase.

6. Procédé selon l'une quelconque des revendications 1 à 5, dans lequel la matière contenant de l'amidon est du grain entier, de préférence du maïs, du blé, de l'orge, ou du sorgho.

7. Procédé selon l'une quelconque des revendications 1 à 6, dans lequel l'amylase ou l'amylase maltogénique est d'origine bactérienne, de préférence une souche du genre *Bacillus,* particulièrement *Bacillus stearothermophilus.*

8. Procédé selon l'une quelconque des revendications 5 à 7, dans lequel l'estérase est une lipase, une phospholipase, ou une cutinase, ou une combinaison de ceux-ci.

9. Procédé selon l'une quelconque des revendications 1 à 8, dans lequel en outre la liquéfaction est réalisée en présence d'enzyme d'oxydation d'acides gras, de préférence une lipoxygenase.

10. Procédé pour la production d'un produit de fermentation, comprenant
(a) la réduction de la taille d'une matière contenant de l'amidon ;
(b) la liquéfaction du produit de l'étape (a) avec au moins une alpha-amylase et au moins une amylase maltogénique telle que définie dans l'une quelconque des revendications 1 à 9 ;
(c) la saccharification de la matière liquéfiée obtenue dans l'étape (b) avec une enzyme générant une source de glucides ; et
(d) la fermentation de la matière saccharifiée en utilisant un microorganisme de fermentation.

11. Procédé selon la revendication 10, dans lequel les étapes c) et d) sont réalisées en tant qu'une étape de saccharification et fermentation simultanée (SFS).

12. Procédé selon l'une quelconque des revendications 10 ou 11, dans lequel l'enzyme générant une source de glucides est une glucoamylase ou une alpha-amylase des mélanges de celles-ci, de préférence dans un mélange d'activité d'alpha-amylase fongique acide (AFAU) par activité de glucoamylase (AGU) (AFAU par AGU) d'au moins 0,1, en particulier d'au moins 0,16, tel que dans la plage allant de 0,12 à 0,50.

13. Procédé selon l'une quelconque des revendications 10 à 12, comprenant en outre la distillation de la matière fermentée.

14. Procédé selon l'une quelconque des revendications 10 à 13, dans lequel ledit microorganisme de fermentation est une levure.

15. Procédé selon l'une quelconque des revendications 10 à 14, dans lequel le produit de fermentation est l'éthanol.
